# EUROPEAN PATENT SPECIFICATION

(11) **EP 2 598 087 B1**
(45) Date of publication and mention of the grant of the patent: **25.03.2015**
(21) Application number: 11755127.5
(22) Date of filing: 27.07.2011
(51) Int. Cl.: A61F 5/01

(54) **SUPPORT FOR HUMAN JOINTS**
STÜTZE FÜR MENSCHLICHE GELENKE
SUPPORT POUR ARTICULATIONS HUMAINES

(30) Priority: 28.07.2010 IT MI20101390
(43) Date of publication of application: 05.06.2013
(73) Proprietor: Indaco S.r.l., 85020 Filiano PZ (IT)
(72) Inventor: FRANGI, Roberta, 21020 Buguggiate VA (IT); FRANGI, Massimo, 57028 Suvereto LI (IT)
(74) Representative: Perani, Aurelio
(86) International application number: PCT/IB2011/053342
(87) International publication number: WO 2012/014164

(56) References cited:
- EP-A1- 0 522 484
- US-A- 4 361 142
- US-A- 4 844 057
- US-A- 5 456 268
- US-A- 5 683 353
- US-A1- 2002 107 462
- US-A1- 2006 106 328

## Description

The present invention relates to a support for human joints, in particular to be used in a knee splint in order to limit the bending movements of a knee. The invention also relates to a knee splint which includes such a support for joints.

It is known in the art to use knee splints for limiting possible bending movements of a knee, for example in the course of rehabilitation of the knee after an accident or a surgical intervention.

Knee splints of this type typically comprise a tubular band which wraps around the knee and the parts of the leg adjacent to the knee. The band comprises pockets on opposite sides with respect to the knee, in each of which is received an articulated support for the joint. The articulated supports according to the prior art comprise a first rod and a second rod connected to each other by means of one or more hinges.

In use, the first rod of the support remains substantially solidly attached to the thigh of the user, whilst the second rod is substantially solidly attached to the tibia of the user.

The articulated supports comprise locking means for limiting the reciprocal rotation possible between the two rods and, consequently, also the maximum bending permitted to the knee supported by the knee splint.

According to one prior art, the locking means are provided by small abutment blocks which are fixed to the rods of the support and/or to the articulation means in order to prevent the reciprocal rotational movement of the rods beyond certain permitted angles. The abutment blocks comprise abutment surfaces on which abut the movable parts of the articulated support on reaching the maximum angle of rotation.

This type of prior art, described for example in US-A 4 844 057, is subject to a series of problems.

The loads which are transmitted by the rods to the abutment blocks are relatively high, in relation to the dimensions of the parts and of the abutment surfaces of the blocks themselves. This results in high localized stresses, with the risk of premature breakage of the support, or wear.

Moreover, in order to be able to adjust the maximum angle of rotation permitted by the support, it is necessary to replace the abutment blocks with other blocks of a different size and shape. This makes adjustment laborious and entails the need to stock a plurality of replacement abutment blocks.

Moreover, the locking of the rotation, being imposed by an abutment surface, is rather brusque, and may prove unpleasant or even dangerous for the user of the support.

According to a different technology, illustrated for example in EP-A 0 522 484, the means for limiting the reciprocal angular displacement of the articulated supports comprise a pair of flexible cables connected, with the interposition of respective helical springs, to the rods which form the articulated support. For each of the flexible cables, one end is fixed in a stable manner to the first of the rods while the other end, after passing through a small threaded tube, is provided with a small plate. Coaxially with each of the flexible cables, the helical spring is positioned between the end of the threaded tube and the end plate.

The threaded tube is axially displaceable by means of a collar mounted thereupon and on the rod so as to be able to rotate with respect to the latter without however being displaced longitudinally.

The end of each threaded tube determines an abutment position that can be adjusted by means of the collar against which the plate of the corresponding flexible cable abuts when the cable is pulled counter to the helical spring during the use of the articulated support.

The known device illustrated in EP-A 0 522 484 has numerous drawbacks during use because, for example, it does not prove easy to determine the magnitude of the angular displacement to be permitted to the rods of the support, this involving an operation to be carried out on the adjustment collar of the threaded tube and in addition because, after the choice of a specific abutment position has been made for the plate connected to the flexible cable, during use of the device it may occur that, even inadvertently, the collar is moved and that therefore the articulated support loses its adjustment, with possible damage to the limbs to which the support is fitted.

In view of the state of the art described, it is an aim of the present invention to provide a support for joints which is resistant to wear, and avoids the occurrence of high localized stresses in the support itself.

A further aim of the invention is to provide a support for joints which can be adjusted easily and rapidly, even when the splint is being worn.

Another aim of the invention is to provide a support which permits gradual, or damped, locking of the rotation between the rods.

According to the presen invention, this aim is achieved by means of a support for human joints as defined in claim 1.

The features and advantages of the present invention will become clear from the following detailed description of some forms of practical embodiment, provided by way of non-limiting example with reference to the attached drawings, in which:
- Figure 1 shows a perspective view of a knee splint which comprises joint supports according to the invention;
- Figure 2 shows an exploded view of the rods and of the articulation means of the support;
- Figure 3 shows a perspective view of a support in a substantially extended position;
- Figure 4 shows a view similar to the previous view, with the support in the bent position;
- Figure 5 shows an enlarged view of the cable return means;
- Figure 6 shows an enlarged view of the cable fixing means;
- Figure 7 shows a perspective view of a modified form of embodiment of the support of Figure 3;
- Figure 8 shows a perspective view of the form of embodiment of Figure 7, viewed from the opposite side with respect to that of Figure 7.

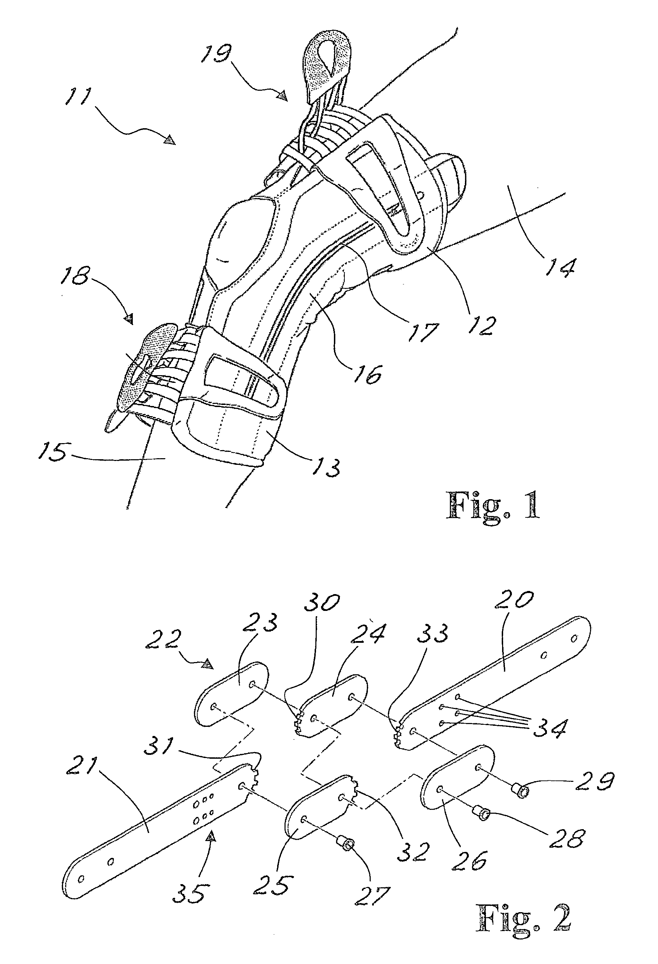

Figure 1 shows a knee splint 11, comprising a first tubular portion 12 intended to wrap around the user's thigh 14 and a second tubular portion 13 intended to wrap around the lower part of the leg, below the knee.

The knee splint also comprises closure means 18, 19, intended to lock the portions 13 and 12 respectively on the leg parts 15 and 14, fixing the tubular band on the leg itself.

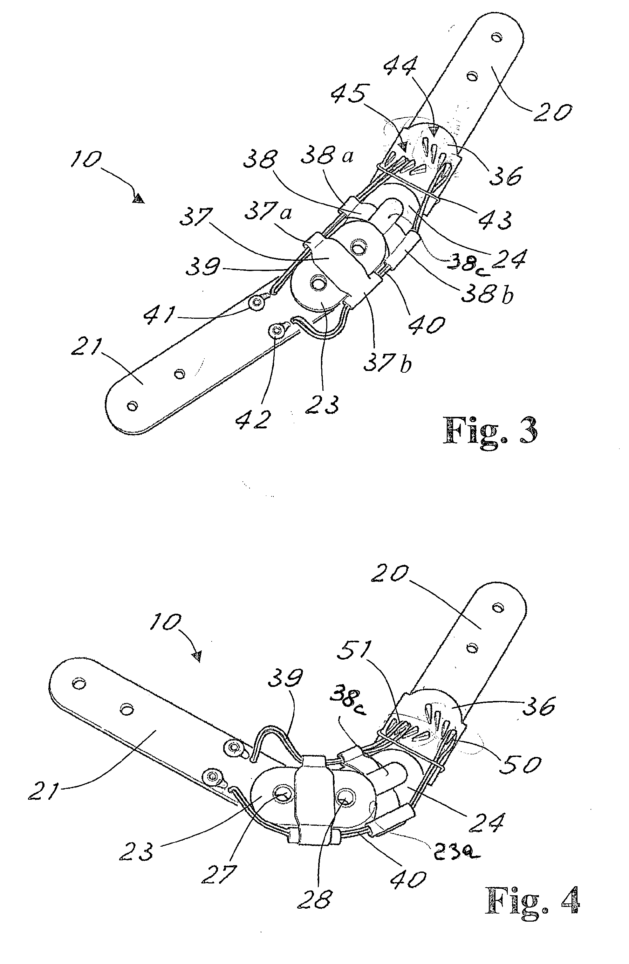

The knee splint also comprises an elongate pocket 16, disposed in the longitudinal direction of the knee splint and extending from the portion 12 to the portion 13 of the knee splint. The pocket 16 is suitable for housing a support 10, of the type illustrated in Figures 3 and 4. The pocket 16 is accessible by opening a zip fastener 17, which extends along the pocket itself. By opening the zip fastener 17 it is possible to gain access to the support 10, for example to adjust or replace it.

The pocket 16 is positioned in such a way as to remain, in use, on the lateral or medial side of the user's knee. On the opposite side of the knee, the knee splint may be provided with another, similar pocket, intended to receive a further support 10.

The support 10 comprises a first rod 20 suitable for being attached substantially solidly to a first human body part, in the exemplary embodiment described, to the thigh 14 of a user.

The support 10 further comprises a second rod 21 suitable for being attached substantially solidly to a second human body part, in the exemplary embodiment described, to the lower part 15 of the user's leg.

The attachment of the rods 20, 21 to the user's leg is effected by means of the portions 12, 13 of the knee splint.

According to the invention, the first and the second rod 20, 21 are connected to each other via articulation means 22 suitable for permitting reciprocal rotation of the two rods about at least one rotation pin. The rods 20, 21 are connected to each other at respective ends.

In a preferred embodiment, the rods 20 and 21 are flattened in shape.

The rods 20, 21 are elongate in form. Preferably, the rods 20, 21 may have a length greater than 10 cm, and more preferably greater than 15 cm. The width of the rods 20, 21 may be between 10% and 25% of their length.

In a preferred embodiment of the invention, the articulation means 22 comprise three pins 27, 28, 29 and at least two connecting elements 23 and 24 connected between the two rods 20, 21 in sequence by means of the pins 27, 28, 29.

This makes it possible to form a more gentle bend between the rod 20 and the rod 21, with a certain radius of curvature which approximates to that of the human joint to be supported.

The rotation pins 27, 28, 29 of the articulation means are normal to the plane in which the rods 20, 21 extend.

Preferably, the articulation means 22 also comprise the two connecting elements 25, 26, which are assembled rigidly and parallel respectively to the connecting elements 23 and 24.

The connecting elements 23-26 are preferably formed by flattened rods. Each connecting element 23-26 is provided with two holes at the opposite ends. The flattened rods 23-26, when assembly is completed, are arranged substantially in a plane parallel to the plane in which the rods 20, 21 extend.

The connecting elements 23 and 25 are hinged to the rod 21 by means of the pin 27 and to the elements 24 and 26 by means of the pin 28. The elements 24 and 26 are hinged at one end to the elements 23 and 25, and at the opposite end to the rod 20.

The rod 21 is interposed with one of its ends between the element 23 and the element 25. Similarly, the rod 20 is interposed between the element 24 and the element 26.

The attachment means 22 have toothed couplings between the connecting elements 23-26 and the rods 20, 21 such as to impart a single degree of freedom to the system formed by the rod 20, rod 21 and securing means. Preferably, the toothed couplings impart - in any possible position assumed by the support 10 - reciprocal inclinations of equal magnitude between the various components 20-26 that are adjacent to one another. In other words, in a preferred form of the invention, the angle between the rod 20 and the connecting element 24 is always equal to the angle between the connecting element 24 and the connecting element 23, and also to the angle between the connecting element 23 and the rod 21.

This makes it possible to guide the bending of the joint to be supported in a desired manner, causing the rods 20, 21 and the articulation means 22 to form a trajectory without sharp angles.

Preferably, the element 24 has at one end a toothed portion 30 which meshes with a toothed portion 31 present on the end of the rod 21. The element 25 has at one end a toothed portion 32 which meshes on a toothed portion 33 present at one end of the rod 20. The toothed couplings 30-33 impart a single degree of freedom to the assembly formed by the components 20-26, as described previously.

According to the invention, the support 10 comprises locking means for limiting the rotation between the two rods in at least one direction of rotation. The locking means comprise at least one flexible cable 40.

At one of its ends the flexible cable 40 can be fixed to a rod 21 or to the articulation means 22, and at the opposite end to the other rod 20 or to the articulation means 22.

According to the invention, the cable 40 is under tension when the two rods 20, 21 reach a maximum reciprocal inclination permitted by the locking means. To a great extent, the length of the cable 40 limits the reciprocal rotation of the two rods 20, 21 in an angular direction, when it is completely under tension and works in traction. This makes it possible to avoid having abutment surfaces on which high pressures are exerted when the support reaches the position of maximum inclination permitted. In addition, the cable which is of a certain length, can extend slightly owing to its natural resilience, avoiding abrupt locking of the rotation when the rods reach the maximum inclination; damped locking of the rotation is thus obtained.

Preferably, as illustrated in Figures 3 and 4, the cable 40 can be fixed at one end to the rod 20 and at the opposite end to the rod 21.

This makes it possible to cause the cable 40 to follow an outline with a certain radius of curvature, defined by the articulation means 22 which has a plurality of pins. In this way, it is possible to reduce the wear and stresses on the cable itself.

Preferably, the support comprises two cables 39, 40, substantially similar, and each intended to limit the reciprocal rotation of the rods 20, 21 in angular directions opposed to each other. In particular, the cable 40 limits the bending of the knee, while the cable 39 limits the maximum extension of the joint. In general, the cable 40 is under tension with the rods bent at a certain angle between them other than zero, while the cable 39 is under tension when the rods are substantially aligned with each other.

Preferably, the cable 40 imposes a limit on rotation in the direction of bending (or flexing) of the support 10 which is between 0° and 90°. The cable 39 imposes a limit on rotation of the rods in the direction of extension of between 0° and 50°.

The cable 40 can be fixed to the rod 21 via fixing means 35 and 42, indicated in Figures 2 and 3 and which, in the context of the present description are also defined as second fixing means.

In particular, the cable 40 is fixed by passing through two holes 35 provided on the rod 21 and via a washer 42 fixed to the rod 21 by a suitable pin.

Similarly, the cable 39 can be fixed to the rod 21 via holes 35, and a washer 41 attached to the rod 21 by means of a respective second pin which, within the scope of the present description are also defined as fourth fixing means.

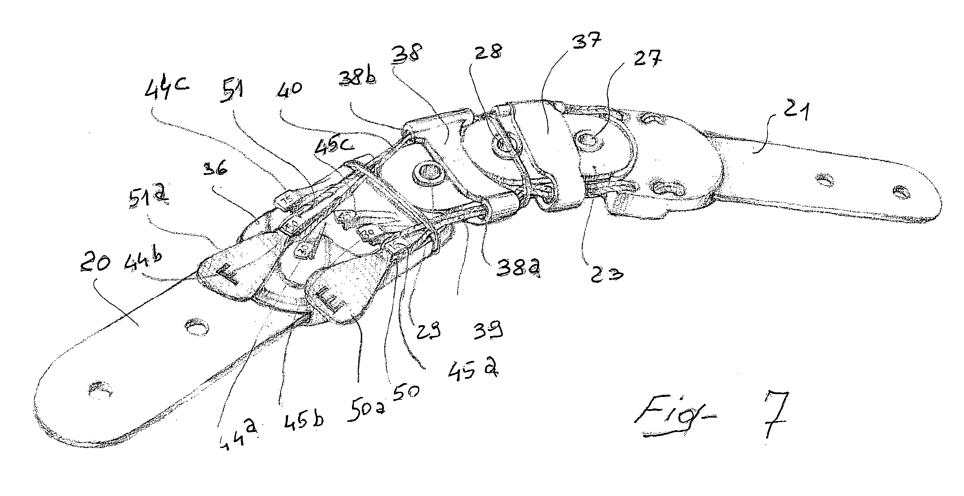

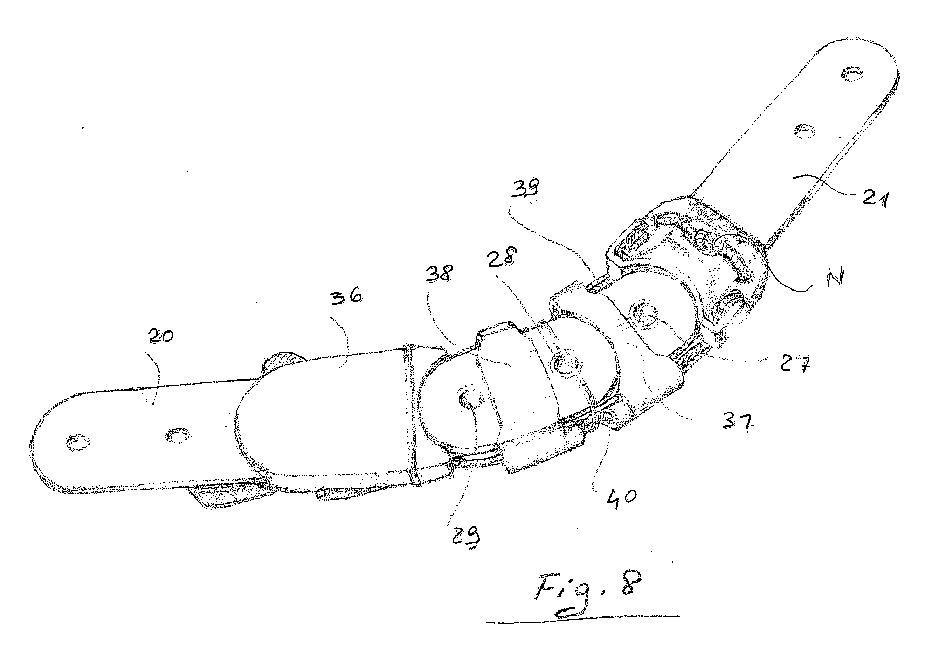

Preferably, the cables 39, 40 form a loop at their end fixed to the rod 21, which winds around the pin for fixing the respective washer 41, 42 to the rod 21. It is to be understood that fixing means of another type may also be used for fixing the cables 39, 40 to the rod 21 such as, for example, those illustrated in Figures 7 and 8 in which the ends of the cables are connected to each other forming a knot N.

According to a particularly preferred embodiment of the invention, the fixing means 44 which fix the other end of the cable 40 are adjustable, in order to be able to vary the anchorage position of the end of the cable with respect to the rod 20.

This makes it possible to vary the maximum inclination that can be reached by the rods 20, 21, enabling the maximum bending of the human joint supported by the support 10 to be adjusted. This possibility is very important during the rehabilitation of a patient, since in the course of time it should be possible for a different freedom of movement of the joint to be permitted.

The possibility of adjusting the fixing position of the cable 40 enables the maximum inclination of the rods to be adjusted without having to replace any component of the support, as happened in the prior art, where it was necessary to have available a set of abutment blocks of varied dimension to be inserted into the support or to intervene in order to adjust the collar of the abutment device. Adjustment therefore becomes much easier and more convenient.

In the preferred embodiment shown in the drawing, the fixing means 44, also defined as first fixing means, comprise a plurality of hooks 44a, 44b, 44c; one end of the cable 40 forms a loop 50 suitable for being selectively engaged with one of the hooks (Fig. 4). To each hook there corresponds a different maximum inclination that can be reached by the rods 20, 21, for example 30°, 50° or 75°.

Preferably, the hooks 44a, 44b, 44c are distributed in sequence in a direction substantially transverse to the longitudinal extent of the rod 20.

The hooks themselves 44a, 44b, 44c are formed on a larger face of the rod 20. Preferably the hooks are formed on a body 36 of flattened form which can be fixed on a face of the rod 20.

The support also comprises adjustable fixing means 45, also defined as third fixing means for an end of the cable 39. The fixing means 45 may also advantageously comprise a plurality of hooks 45a, 45b, 45c, similar to the hooks 44 previously described, formed on the body 36 and arranged in sequence in a direction substantially transverse to the longitudinal extent of the rod 20.

To each hook 45a, 45b, 45c there corresponds a different degree of inclination between the rods 20 and 21, for example 15°, 30° or 45°, it therefore being possible to adjust also the maximum alignment permitted.

Advantageously, the hooks 44 have inclinations different from one another with respect to the longitudinal axis of the rod 20, or, an increasing inclination with respect to that axis as the distance between the hook and a lateral edge of the rod 21 increases. This makes it possible to have a hook substantially oriented always in the direction of the respective cable 40 to be attached, increasing the resistance of the hooks to the tensile stresses exerted by the cable.

The hooks 45 are in a similar arrangement to the hooks 44, also with regard to inclination.

Preferably, the support 10 comprises return means 37, 38 which, in an intermediate position between their two ends, cause the cables 39 and 40 to pass into at least one passage point arranged at a distance from the axis of rotation of at least one pin of the articulation means 22.

Preferably, the return means 37, 38 are mounted on the connecting elements 23, 24.

In an advantageous embodiment, the return means comprise at least one element 37 mounted on the connecting element 23 and an element 38 mounted on the connecting element 24.

Preferably the elements 37, 38 each form eyelets which provide a passage point for the cables 39 and 40.

Advantageously, the elements 37, 38 are annular in shape, in order to be slipped respectively onto the connecting element 23 and 24.

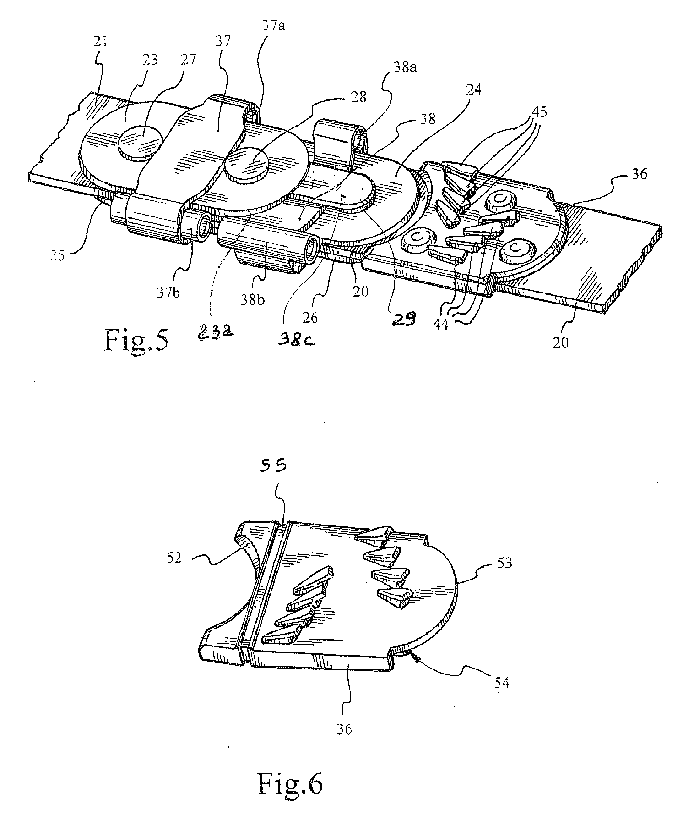

As is clearly shown in Figure 5, the annular element 37 surrounds the connecting element 23 and the connecting element 25 solidly connected thereto.

The annular element 38 surrounds the connecting element 24 and the connecting element 26 and is equipped with an appendage 38c which, in the form of embodiment of Figures 3, 4 and 5, engages with the pin 29 for connection between the rod 20 and the connecting elements 24 and 26.

Each annular element 37, 38 forms two eyelets 37a, 37b, 38a and 38b disposed on opposite sides of the connecting elements 23, 24.

More generally, it is advantageous for the passage points defined by the return means to be in proximity to the straight lines tangent to the lateral edges of the rods 20, 21. In this way, the cable passes at a maximum distance from the rotation pins, and it is possible to vary substantially the angles of maximum rotation between the rods even with a small displacement of the fixing point for the end of the cable.

Preferably, the return means associated with each cable 39, 40 form a passage point for the cable solidly connected to a connecting element 23, and a passage point solidly connected to a second connecting element 24. The cables can thus be made to follow as rounded a trajectory as possible, avoiding sudden changes of direction which could damage the cable or cause it to wear more rapidly.

Advantageously, the annular element 37 is locked with respect to the connecting element 23 since it remains engaged between the heads of the two pins 27 and 28 (Fig. 5) and the annular element 38 is not free to slide with respect to the connecting element 24 because the appendage 38c is engaged with the head of the pin 29.

However, according to the form of embodiment illustrated in Figures 7 and 8, the appendage 38c is absent and the annular element 38 remains locked between the arcuate profile 23a of the connecting element 23 and the head of the pin 29.

Figure 6 shows an embodiment of the body 36 in the form of a small plate, on which are formed the adjustable fixing means for the ends of the cables 39 and 40. The body 36 advantageously has two straight sides parallel to the edges of the rod 21, and two curved sides 52, 53, respectively concave and convex. On the face to the rear of that on which the hooks 44, 45 are formed, the body 36 has means 54 for engagement on the rod 20.

With reference to the form of embodiment of Figures 7 and 8, the engagement means 54 are absent because the body 36 is provided in one piece with the end of the rod 20.

In proximity to the side which, in use, faces towards the articulation means, the body has a groove 55, transverse to the axis of the rod 20 and suitable for receiving a cable 43 (shown in Figure 3). The cable 43 surrounds the body 36 and the rod 20 and, in use, also the cables 39, 40, in such a way as to prevent the loops 50 and 51 of the cables 39, 40 from being disengaged from the respective hooks 44, 45 when the cables are not under tension.

The cable 43 may be made resilient and, when it is wished to adjust the fixing point of the cables 39, 40, can easily be extended so that the loops 50, 51 can be moved.

The latter, according to the form of embodiment of Figures 7 and 8, are provided with respective tongues 50a and 50b which make it easier for the user to grip with the fingers. The tongues may also each be provided with the indication of the function of the corresponding cable. For example, they may be provided with an F for the cable intended for adjustment of the flexion, and with an E for the cable intended for the adjustment of the extension of the device.

Advantageously, the cables 39 and 40 may be made of very high molecular weight polyethylene. For example, "Dyneema" cables may be used.

At this point it becomes clear that the objects of the present invention have been achieved.

In particular, a support for human joints is provided, in particular for the knees, which is particularly resistant to wear, and avoids the occurrence of high localized stresses when the limit of rotation imposed by the support is reached. Moreover, the adjustment of the maximum angles permitted, generally about 44°, is particularly easy and rapid, without requiring the replacement of locking elements in the support itself.

Naturally, a person skilled in the art, for the purpose of fulfilling contingent and specific requirements, may apply numerous modifications and variants to the configurations described above, all however included within the scope of protection of the invention as defined by the following claims.

## Claims

1. A support (10) for human joints comprising a first rod (20) intended to be attached substantially solidly to a first human body part and a second rod (21) intended to be attached substantially solidly to a second human body part, the first and second rods being connected to each other via articulation means (22) suitable for permitting reciprocal rotation of the two rods (20, 21) about at least one rotation pin (27), the support comprising locking means for limiting the rotation between the two rods in at least one angular direction, the locking means comprising at least one flexible cable (40), the support (10) comprising first fixing means (44) for anchoring a first end of the cable (40) to the first rod (20) and second fixing means (42) for fixing a second end of the cable (40) to the second rod (21), the cable (40) being under tension when the two rods (20, 21) reach a maximum reciprocal inclination permitted by the length of the cable, **characterized in that** the first fixing means (44) comprise a plurality of hooks (44a, 44b, 44c), said first end of at least one flexible cable (40) forms a loop (50) suitable for being engaged selectively with one of the hooks of the plurality of hooks (44a, 44b, 44c) in order to be able to vary the anchorage position of the first end of the cable (40) with respect to the first rod (20).

2. A support according to claim 1, **characterized in that** the locking means comprise a second flexible cable (39) and the support (10) comprises third fixing means (45) for anchoring a first end of the second cable (39) to the first rod (20) and fourth fixing means (41) for fixing a second end of the second cable (39) to the second rod (21), the second cable (39) being intended to limit the reciprocal rotation between the two rods (20, 21) in an opposite angular direction with respect to the angular direction limited by the first flexible cable (40), the third fixing means (45) of the second cable (39) comprising a plurality of hooks (45a, 45b, 45c), the first end of said second flexible cable (39) forming a loop (51) suitable for being engaged selectively with one of the hooks of the plurality of hooks (45a, 45b, 45c) in order to be able to vary the anchorage position of the first end of the second cable (39) with respect to the first rod (20).

3. A support according to claim 1 or 2, **characterized in that** the plurality of hooks (44a, 44b, 44c, 45a 45b, 45c) comprise hooks, each of which is integral with the first rod (20).

4. A support according to any one of the preceding claims, **characterized in that** the plurality of hooks (44a, 44b, 44c, 45a 45b, 45c) comprise hooks arranged in sequence in a direction substantially transverse to the longitudinal extent of the rod (20) on which they are provided.

5. A support according to one or more of the preceding claims, **characterized in that** it comprises return means (37, 38) which, in a position intermediate between the two ends of the cable (40) and/or of the second cable (39), cause the cable (40) and/or the second cable (39) to pass into at least one passage point disposed at a distance from the axis of rotation of the rotation pin (27).

6. A support according to one or more of the preceding claims, **characterized in that** the articulation means (22) comprise three rotation pins (27-29) and two connecting elements (23, 24) articulated in sequence by means of the three pins (27-29) in order to connect the two rods (20, 21).

7. A support according to one or more of the preceding claims, **characterized in that** the return means (37, 38) are mounted on the connecting elements (23, 24).

8. A support according to one or more of the preceding claims, **characterized in that** the return means comprise two annular elements (37, 38), each slipped onto a respective connecting element (23, 24), the annular elements forming at least one return eyelet (37a, 37b, 38a, 38b) for a cable (39, 40).

9. A knee splint comprising a first tubular portion (12) suitable for being slipped onto the user's thigh, a second tubular portion (13) suitable for being slipped onto the lower portion of the leg and a knee which connects the two tubular portions, the knee splint comprising at least one support (10) according to one or more of the preceding claims, the support being disposed, in use, on at least one side of the knee splint with the rods (20, 21) each rigidly connected to one of the tubular portions (12, 13) and the articulation means at knee height, the two rods (20, 21) being substantially aligned respectively with the first and the second tubular portion (14, 15).

## Patentansprüche

1. Stütze (10) für menschliche Gelenke, die eine erste Stange (20), die dazu vorgesehen ist, im Wesentlichen fest an einem ersten menschlichen Körperteil angebracht zu werden, und eine zweite Stange (21) umfasst, die dazu vorgesehen ist, im Wesentlichen fest an einem zweiten menschlichen Körperteil angebracht zu werden, wobei die erste und die zweite Stange durch Gelenkmittel (22) miteinander verbunden sind, die geeignet sind, die gegenseitige Verdrehung der zwei Stangen (20, 21) um mindestens einen Drehbolzen (27) zu ermöglichen, wobei die Stütze Arretierungsmittel zum Begrenzen der Verdrehung zwischen den Stangen in mindestens einer Winkelrichtung umfasst, wobei die Arretierungsmittel mindestens ein flexibles Seil (40) umfassen, wobei die Stütze (10) erste Befestigungsmittel (44) zum Verankern eines ersten Endes des Seils (40) an der ersten Stange (20) und zweite Befestigungsmittel (42) zum Befestigen eines zweiten Endes des Seils (40) an der zweiten Stange (21) umfasst, wobei das Seil (40) unter Spannung steht, wenn die zwei Stangen (20, 21) eine maximale Neigung zueinander erreichen, die von der Länge des Seils zugelassen wird, **dadurch gekennzeichnet, dass** die ersten Befestigungsmittel (44) eine Vielzahl von Haken (44a, 44b, 44c) umfassen, wobei das erste Ende von mindestens einem flexiblen Seil (40) eine Schlaufe (50) bildet, die selektiv mit einem der Haken der Vielzahl von Haken (44a, 44b, 44c) in Eingriff gebracht werden kann, damit es möglich ist, die Verankerungsposition des ersten Endes des Seils (40) gegenüber der ersten Stange (20) zu verändern.

2. Stütze nach Anspruch 1, **dadurch gekennzeichnet, dass** die Arretierungsmittel ein zweites flexibles Seil (39) umfassen und die Stütze (10) dritte Befestigungsmittel (45) zum Verankern eines ersten Endes des zweiten Seils (39) an der ersten Stange (20) und vierte Befestigungsmittel (41) zum Befestigen eines zweiten Endes des zweiten Seils (39) an der zweiten Stange (21) umfasst, wobei das zweite Seil (39) dazu vorgesehen ist, die gegenseitige Verdrehung zwischen den zwei Stangen (20, 21) in einer Winkelrichtung zu begrenzen, die der Wickelrichtung entgegengesetzt ist, die durch das erste flexible Seil (40) begrenzt wird, wobei die dritten Befestigungsmittel (45) des zweiten Seils (39) eine Vielzahl von Haken (45a, 45b, 45c) umfassen, wobei das erste Ende des zweiten flexiblen Seils (39) eine Schlaufe (51) bildet, die selektiv mit einem der Haken der Vielzahl von Haken (45a, 45b, 45c) in Eingriff gebracht werden kann, damit es möglich ist, die Verankerungsposition des ersten Endes des zweiten Seils (39) gegenüber der ersten Stange (20) zu verändern.

3. Stütze nach Anspruch 1 oder 2, **dadurch gekennzeichnet, dass** die Vielzahl von Haken (44a, 44b, 44c, 45a, 45b, 45c) Haken umfasst, von denen jeder integral mit der ersten Stange (20) ist.

4. Stütze nach einem der vorhergehenden Ansprüche, **dadurch gekennzeichnet, dass** die Vielzahl von Haken (44a, 44b, 44c, 45a, 45b, 45c) Haken umfasst, die nacheinander in einer Richtung angeordnet sind, die im Wesentlichen quer zur Längserstreckung der Stange (20) ist, auf der sie vorgesehen sind.

5. Stütze nach einem oder mehreren der vorhergehenden Ansprüche, **dadurch gekennzeichnet, dass** sie Rückführmittel (37, 38) umfasst, die in einer Zwischenposition zwischen den zwei Enden des Seils (40) und/oder des zweiten Seils (39) bewirken, dass das Seil (40) und/oder das zweite Seil (39) in mindestens einen Durchgangspunkt gelangt, der sich in einem Abstand von der Drehachse des Drehbolzens (27) befindet.

6. Stütze nach einem oder mehreren der vorhergehenden Ansprüche, **dadurch gekennzeichnet, dass** die Gelenkmittel (22) drei Drehbolzen (27-29) und zwei Verbindungselemente (23, 24), die mittels der drei Drehbolzen (27-29) nacheinander gelenkig verbunden sind, umfassen, um die zwei Stangen (20, 21) zu verbinden.

7. Stütze nach einem oder mehreren der vorhergehenden Ansprüche, **dadurch gekennzeichnet, dass** die Rückführmittel (37, 38) auf die Verbindungselemente (23, 24) montiert sind.

8. Stütze nach einem oder mehreren der vorhergehenden Ansprüche, **dadurch gekennzeichnet, dass** die Rückführmittel zwei ringförmige Elemente (37, 38) umfassen, die jeweils auf ein entsprechendes Verbindungselement (23, 24) geschoben sind, wobei die ringförmigen Elemente mindestens eine Rückführöse (37a, 37b, 38a, 38b) für ein Seil (39, 40) bilden.

9. Knieschiene, die einen ersten röhrenförmigen Abschnitt (12), der über den Oberschenkel des Benutzers geschoben werden kann, einen zweiten röhrenförmigen Abschnitt (13), der über den unteren Teil des Beins geschoben werden kann, und ein Kniestück umfasst, das die zwei röhrenförmigen Abschnitte verbindet, wobei die Knieschiene mindestens eine Stütze (10) nach einem oder mehreren der vorhergehenden Ansprüche umfasst, wobei die Stütze beim Gebrauch auf mindestens einer Seite der Knieschiene angeordnet ist, wobei die Stangen (20, 21) jeweils starr mit einem der röhrenförmigen Abschnitte (12, 13) verbunden sind und sich die Gelenkmittel auf der Höhe des Kniestücks befinden, wobei die zwei Stangen (20, 21) im Wesentlichen mit dem ersten beziehungsweise zweiten röhrenförmigen Abschnitt (14, 15) gefluchtet sind.

## Revendications

1. Support (10) pour articulations humaines comprenant une première tige (20) destinée à être fixée sensiblement solidement à une première partie de corps humain et une deuxième tige (21) destinée à être fixée sensiblement solidement à une deuxième partie de corps humain, les première et deuxième tiges étant reliées entre elles par l'intermédiaire de moyens d'articulation (22) adaptés pour permettre une rotation réciproque des deux tiges (20, 21) autour d'au moins une broche de rotation (27), le support comprenant des moyens de blocage pour limiter la rotation entre les deux tiges dans au moins une direction angulaire, les moyens de blocage comprenant au moins un câble flexible (40), le support (10) comprenant des premiers moyens de fixation (44) pour fixer une première extrémité du câble (40) à la première tige (20) et des deuxièmes moyens de fixation (42) pour fixer une deuxième extrémité du câble (40) à la deuxième tige (21), le câble (40) étant sous tension quand les deux tiges (20, 21) atteignent une inclinaison réciproque maximale permise par la longueur du câble, **caractérisé en ce que** les premiers moyens de fixation (44) comprennent une pluralité de crochets (44a, 44b, 44c),ladite première extrémité d'au moins un câble flexible (40) forme une boucle (50) adaptée pour être engagée de manière sélective avec un des crochets de la pluralité de crochets (44a, 44b, 44c) de manière à être en mesure de changer la position de fixation de la première extrémité du câble (40) par rapport à la première tige (20).

2. Support selon la revendication 1, **caractérisé en ce que** les moyens de fixation comprennent un deuxième câble flexible (39) et le support (10) comprend des troisièmes moyens de fixation (45) pour fixer une première extrémité du deuxième câble (39) à la première tige (20) et des quatrièmes moyens de fixation (41) pour fixer une deuxième extrémité du deuxième câble (39) à la deuxième tige (21), le deuxième câble (39) étant destinée à limiter la rotation réciproque entre les deux tiges (20, 21) dans une direction angulaire opposée par rapport à la direction angulaire limitée par le premier câble flexible (40), les troisièmes moyens de fixation (45) du deuxième câble (39) comprenant une pluralité de crochets (45a, 45b, 45c), la première extrémité dudit deuxième câble flexible (39) formant une boucle (51) adaptée pour être engagée de manière sélective avec un des crochets de la pluralité de crochets (45a, 45b, 45c) de manière à être en mesure de changer la position de fixation de la première extrémité du deuxième câble (39) par rapport à la première tige (20).

3. Support selon la revendication 1 ou 2, **caractérisé en ce que** la pluralité de crochets (44a, 44b, 44c, 45a, 45b, 45c) comprend des crochets, chacun desquels est d'une seule pièce avec la première tige (20).

4. Support selon l'une quelconque des revendications précédentes, **caractérisé en ce que** la pluralité de crochets (44a, 44b, 44c, 45a, 45b, 45c) comprend des crochets agencés en séquence dans une direction sensiblement transversale à l'extension longitudinale de la tige (20) sur laquelle ils sont disposés.

5. Support selon une ou plusieurs des revendications précédentes, **caractérisé en ce qu'**il comprend des moyens de retour (37, 38) qui, dans une position intermédiaire entre les deux extrémités du câble (40) et/ou du deuxième câble (39), forcent le câble (40) et/ou le deuxième câble (39) à passer dans au moins un point de passage disposé à une distance de l'axe de rotation de la broche de rotation (27).

6. Support selon une ou plusieurs des revendications précédentes, **caractérisé en ce que** les moyens d'articulation (22) comprennent trois broches de rotation (27-29) et deux éléments de liaison (23, 24) articulés en séquence au moyen des trois broches (27-29) de manière à relier les deux tiges (20, 21).

7. Support selon une ou plusieurs des revendications précédentes, **caractérisé en ce que** les moyens de retour (37, 38) sont montés sur les éléments de liaison (23, 24).

8. Support selon une ou plusieurs des revendications précédentes, **caractérisé en ce que** les moyens de retour comprennent deux éléments annulaires (37, 38), chacun glissé sur un élément de liaison respectif (23, 24), les éléments annulaires formant au moins un oeillet de retour (37a, 37b, 38a, 38b) pour un câble (39, 40).

9. Attelle de genou comprenant une première partie tubulaire (12) adaptée pour être glissée sur la cuisse de l'utilisateur, une deuxième portion tubulaire (13) adaptée pour être glissée sur la partie inférieure de la jambe et un genou qui relie les deux parties tubulaires, l'attelle de genou comprenant au moins un support (10) selon une ou plusieurs des revendications précédentes, le support étant disposé, durant l'utilisation, sur au moins un côté de l'attelle de genou avec les tiges (20, 21), chacune connectée rigidement à une des parties tubulaires (12, 13) et aux moyens d'articulation à hauteur du genou, les deux tiges (20, 21) étant sensiblement alignées respectivement avec la première et la deuxième partie tubulaire (14, 15).
